# EUROPEAN PATENT SPECIFICATION

(11) **EP 4 132 394 B1**
(45) Date of publication and mention of the grant of the patent: **03.09.2025**
(21) Application number: 21717830.0
(22) Date of filing: 07.04.2021
(51) Int. Cl.: A61B 17/70, A61B 17/80, A61B 17/86, A61B 17/84, A61F 2/30, A61F 2/44

(54) **FENESTRATED BONE ANCHOR**
FENESTRIERTER KNOCHENANKER
ANCRAGE OSSEUX FENÊTRÉ

(30) Priority: 08.04.2020 CH 4292020
(43) Date of publication of application: 15.02.2023
(73) Proprietor: WoodWelding AG, 6362 Stansstad (CH)
(72) Inventor: MÜLLER, Andrea, 8408 Winterthur (CH)
(74) Representative: Frei Patent Attorneys
(86) International application number: PCT/EP2021/059113
(87) International publication number: WO 2021/204908

(56) References cited:
- EP-A1- 3 207 901
- EP-A1- 3 607 914
- EP-A1- 3 616 634
- US-A1- 2014 052 258
- US-A1- 2015 216 573
- US-A1- 2016 058 480
- US-A1- 2017 354 504

## Description

### FIELD OF THE INVENTION

The invention is in the field of medical technology. It relates to a fenestrated bone anchor, i.e. to a surgical item being suitable for being anchored in live bone tissue of a human or animal patient and comprising a longitudinal cavity extending along an anchor axis from a proximal anchor end towards a distal anchor end, and at least one lateral channel connecting a circumferential anchor surface with the longitudinal cavity.

### BACKGROUND OF THE INVENTION

Fenestrated bone anchors are widely known in the field of human and veterinary surgery. They are suitable in particular for an anchor retention with the aid of a bone cement or a material having thermoplastic properties, wherein the bone cement is pressed in a flowable state into the longitudinal cavity and from there through the lateral channel or channels to contact and possibly augment live bone tissue surrounding the anchor, or wherein the material having thermoplastic properties is positioned in the longitudinal cavity in a solid state, is liquefied therein by application of energy, preferably vibration energy, and, in a liquefied state, is pressed through the lateral channel or channels for achieving a similar effect as achieved with the bone cement. Fenestrated bone anchors can also be used for administering pharmaceutical substances to the bone tissue surrounding the anchor.

Fenestrated bone anchors comprise a shaft and possibly a head. The circumferential shaft surface may comprise any sort of per se known means for retaining the anchor in bone tissue in addition to the possible retention achievable by the above described augmentation. Such retaining means are e.g. threads, teeth, cutting edges, ribs, resilient projections or also osseointegration enhancing surface roughness or surface coatings.

Fenestrated bone anchors are applicable e.g. for stabilizing bone fragments or misaligned bones relative to each other or for fixating soft tissue on bone tissue. Furthermore, they are applicable for fixating artificial items relative to bone tissue, such as e.g. bone plates, interbody fusion devices, intramedullary nails or rods, instrumentation for spine correction or support, and also sutures and cables for various applications. They are also applicable for bone augmentation. Bone anchors to be used in the named or other exemplary applications are simple fixation pins or screws with or without heads, pedicle screws, interference devices, suture anchors, dental implants etc.

EP 3 616 634 concerns screw implants for bone fusion. The screw implants may have a shaft with biomaterial windows into which biomaterial, such as bone graft can be filled. In one embodiment, the biomaterial windows can be in fluid communication with a cannulated opening that serves for receiving a guide wire or other type of instrument to assist in implantation. The diameter of the cannulation is smaller than a width of the biomaterial windows and does not serve for, and would not be suitable for, feeding the biomaterial windows with material. The biomaterial windows have shapes that are elongate, with long sides running parallel to the longitudinal axis between distal and proximal end sections.

The publication WO2010/096942 (publ. European application EP3207901 A1) discloses a system with a plurality of fenestrated bone anchors. In addition to the plurality of fenestrated bone anchors, the disclosed system comprises an interbody fusion device of the stand-alone type, i.e., an interbody implant to be positioned between neighboring vertebral bodies and a plate suitable for retaining the implant in its position. The plate has a plurality of through openings and the bone anchors are adapted to the through openings and suitable for fixating the plate to the neighboring vertebral bodies. The implant and the plate may be two separate items, may be fixed to each other or may form together one integral part. The disclosed bone anchors have the form of fenestrated headed pins. They comprise a longitudinal cavity and lateral channels connecting the longitudinal cavity with the circumferential surface of the pin and they are described to be suitable for being anchored in the bone tissue of the vertebral bodies with the aid of a material having thermoplastic properties. For the anchoring process, an opening is provided in the bone tissue, the fenestrated pin is introduced into the opening, an element of the material having thermoplastic properties is inserted into the longitudinal cavity, energy, in particular vibration energy (in particular ultrasonic vibration energy) is applied to the thermoplastic element for liquefying the material having thermoplastic properties, and simultaneously with the application of the energy, the element of the material having thermoplastic properties is pressed into the cavity for displacing the liquefied material through the lateral channels into the adjacent bone tissue or between the circumferential pin surface and the adjacent bone tissue. For achieving preferred anchorage in the vertebral body, the lateral channels may be situated nearer to the proximal pin end than to the distal pin end (e.g. in the proximal half or in the most proximal third of the pin length) for deposition of the material having thermoplastic properties as near as possible to the cortical bone of the wall of the vertebral body, where the bone tissue has a stronger consistency for being penetrated by the liquefied material than the bone tissue nearer the center of the vertebral body.

### SUMMARY OF THE INVENTION

It is the object of the present invention to provide an improved fenestrated bone anchor which has features as described above and which is particularly suitable for the above-mentioned anchorage with the aid of a material having thermoplastic properties, but is applicable for other applications also. The improvement of the fenestrated bone anchor according to the invention regards in particular the at least one lateral channel, the material flow therethrough and its mechanical effect on the stability of the anchor.

The fenestrated bone anchor achieving the above object comprises a shaft and possibly a head, the shaft having a proximal end, a distal end, and a longitudinal axis and a circumferential surface extending from the proximal end to the distal end, as well as a longitudinal cavity extending in the direction of the axis, if applicable, through the head and from the proximal shaft end towards the distal shaft end. Furthermore, the shaft comprises at least one lateral channel (namely a plurality of lateral channels arranged at a same first distance from the proximal end of the shaft, e.g. two to four lateral channels) extending through a wall of the shaft from the longitudinal cavity to the circumferential surface. This wall has a wall thickness and the lateral channel has a cross section with an axial length and a width smaller than the axial length, wherein the width of the cross section of the lateral channel increases in a distal direction and/or the wall thickness increases gradually in selected ones of directions towards the lateral channel. This means that the cross section of the lateral channel or channels has a pear-shape and that in addition or alternatively the wall between the axial cavity and the circumferential surface has a thickness which is not the same around the longitudinal cavity but is largest at least partially where adjoining the lateral channel and is decreasing gradually in a direction away from the lateral channel.

The named design features make the bone anchor according to the invention particularly suitable for an anchoring process by augmentation with the aid of a material having thermoplastic properties as described briefly further above, for applications in which more proximal augmentation is desired, and for applications in which the proximal anchor end is more rigidly fixated (e.g. in a plate or in cortical bone) than the distal anchor end (e.g. in trabecular bone) and therewith bending load increases from the distal end towards the proximal end. The named mechanical conditions are in particular applicable for anchors used for fixating e.g. a plate on a vertebral body or in a meta- or epiphyseal region of a long bone or also for suture anchors, in which cases the preferred anchorage is as near as possible to the cortex where bone density is highest and where a body of augmenting material can lean on the cortex and so to speak extend the thickness of the cortex.

The lateral channel may have a substantial axial extension of less than 30%, for example less than 25%, for example between 12% and 20% of a length of the bone anchor.

For many embodiments, the lateral channel needs to have a substantial axial extension (length) so that the bone tissue can be homogeneously infiltrated in a large range even if there is substantial quenching behavior, as is for example the case for thermoplastic material when it infiltrates the bone in a liquid state. An infiltration in a large area is advantageous not only for solid anchoring but also for reinforcing the bone tissue. In particular, the design measures of the pear-shaped cross section of the lateral channel and/or the wall thickness increasing towards the lateral channel make it possible to give the lateral channels a cross section guaranteeing satisfactory flow of the liquefied material even when the latter is subject to quenching and still giving the anchor mechanical characteristics which allows it to bear the bending stress even if the lateral channel is arranged in a proximal portion of the anchor. The named design measures result in the anchor being homogeneously stressed over its length at a minimum of notch stress, which is particularly important for anchors subjected to cyclical stressing.

Especially, the shape of the lateral channel may be chosen so that across the length (axial extension) of the lateral channel, the medium stress (comparison stress) is essentially homogeneous along the lateral channel. This allows to the lateral channel to have a substantial axial extension while the load-bearing strength is not substantially reduced. Especially, the ratio of the comparison stress to the material strength, when a certain bending moment is applied upon the bone anchor, may be designed to be essentially constant across the length of the lateral channel. To achieve this, the bending stiffness may be chosen to gradually decrease towards distally. This feature of the bending stiffness increasing towards proximally may be present across a substantial portion of the length of the lateral channel (for example in a middle region excluding a proximal and a distal end, for example across at least 40% or at least 50% of its length for example across at least 40% or at least 50% thereof) and not for example only at the proximal/distal end.

For example, the anchor may be designed for the stress at the rim of the lateral channel (given a certain applied bending moment) to vary along the length of the lateral channel by less than about 30% or less than about 20% or less than about 10% (defined as the difference between the maximum stress and the minimum stress divided by the average stress along the complete length), preferably over half of the length of the lateral channel is situated and even more preferably over the entire length of the lateral channel.

A possible design criterion may be that the so-called polar second moment of area J with respect to the longitudinal axis increases, for example continuously increases, towards proximally across the anchor portion in which the at least one lateral channel is situated, for example for at least a substantial portion of its length (for example in a middle region excluding a proximal and a distal end, for example across at least 40% or at least 50% of its length). Especially, the polar second moment of area J may be such that the value x*J is approximately constant across the anchor portion in which the at least one lateral channel is situated, where x is the distance to the proximal end of the bone anchor. In this, "approximately constant" may be defined as varying by not more than 20% or not more than 10% or even not more than 5%.

Fulfilling this design criterion may be helpful in view of the facts that in use the bone anchor is held proximally by relatively stiff structures, namely the implant (for example bone plate) and the cortical bone tissue, and that the lateral channel may be located at a relatively proximal position, away from the distal end.

A further, possibly additional design criterion may be that the material of the bone anchor, the strength of which according to the above design criterion relating to the second moment of area J generally increases towards proximally, is distributed in a manner that the tress along the rim of the lateral channel does not become too high compared to the stress elsewhere and is relatively homogeneous, as mentioned above.

To this end, one possibility is that the wall thickness increases towards the lateral channel. In addition or as an alternative, the shape of the lateral channel (or, to be precise its mouth in the outer surface of the bone anchor shaft) may have the mentioned pear shape. In this latter way, the stress in the more critical more proximal regions can be somewhat lowered along the rim of the lateral channel in that the stress is more distributed and the regions between the lateral channels take up more stress. For the shape of the mouth of the lateral channel, this means that, in addition or as an alternative to the wall thickness increasing towards the lateral channel, one or more of the following may hold:
- At axial positions that correspond to axial positions of a middle region of the lateral channel, the circumferential width decreases continuously in a proximal direction. The circumferential width may for example decrease along at least 40%, at least 50% or at least 60% of the axial extension (from distal to proximal) of the lateral channel, and/or it may decrease from distal to proximal by at least 5%, at least 10% or at least 15%
- At axial positions that correspond to axial positions of a middle region of the lateral channel, the polar second moment of area J increases continuously in a proximal direction. The polar second moment of area J may for example increase from distal to proximal along at least 30%, 40%, at least 50% or at least 60% of the axial extension of the lateral channel, for example by at least 5%, at least 10%, or at least 15% or 20% and for example at most 50%.
- An average circumferential width of a proximal half of the lateral channel is substantially smaller than an average circumferential width of a distal half of the lateral channel. "Substantially smaller" in this context may mean smaller by at least 5% or by at least 10% or even by at least 20%.
- The sidewalls are not parallel to each other but may approximate towards proximal, not only in a proximal and distal end region, but along a substantial portion of the lateral channel's axial extension. Especially, the sidewalls may be not parallel across at least 40%, at least 50% or at least 60% of the axial extension of the lateral channel.
- The sidewalls of the lateral channel are not parallel to the longitudinal axis but extend essentially straight at an angle of between 1° and 20°, especially between 2° and 10°, in particular between 3° and 8°, for example about 5° to the axis, approximating towards proximal.
- The sidewalls are not parallel to each other in a middle region located in the middle between the proximal end and the distal end of the lateral channel, but the sidewalls may approximate towards proximal.

The longitudinal cavity serves for inserting the material that is pressed into surrounding tissue through the at least one lateral channel. It may be essentially cylindrical, at least distally of the head and poximally of the distal end, for example circularly cylindrical (i.e. circular in cross section). A diameter or average diameter of the longitudinal cavity will be larger than a width of the lateral channel, for example substantially larger than the latter. The average diameter of the longitudinal cavity and/or the diameter of the longitudinal cavity at the position of the lateral channel may for example be between 1.1 times and 3 times the maximum width of the lateral channel (i.e., between 1.1 times and 3 times the circumferential width at the position where it is widest, which may be the case generally towards the distal end in embodiments in which the lateral channel is pear shaped). Especially, it may be between 1.2 times or 1.3 times and 2.5 times or between 1.4 times and 2 times, especially between 1.5 times and 1.9 times and for example about 1.7 times the maximum width.

A typical diameter of the longitudinal cavity may be between 1.5 mm and 4.5 mm, especially between 1.7 mm and 3.7 mm, for example about 3-3.2 mm or between 1.7 mm and 2.2 mm (the latter for cervical applications). More in general, the diameter of the longitudinal cavity may amount to between 40% and 87%, especially between 50% and 82% or between 60% and 80%, for example around 75%-79% of the bone anchor shaft diameter.

A height (axial extension of the lateral channel may be between 1.2 times and 4 times its maximum width, especially between 1.4 times and 3.2 times or between 1.6 times and 2.6 times, for example about 1.8-2.4 times the maximum width.

The distal end of the longitudinal cavity in the bone anchor may be closed. In a variant, it may have comparably small (i.e. radial extension being much smaller than the radial extension of the longitudinal cavity) axial opening for a guide wire or similar. The distal end may have a shape different from circularly symmetrical about the axis of the bone anchor. Rather, the channels may have an inner portion at radial positions within a radius of the longitudinal cavity in addition to having an outer portion formed by the (pear-shaped) exit opening in the wall around the cavity. Thus, the distal end of the longitudinal cavity may be angularly structured so as to direct different portions of the material to the different exit openings.

Such angular structure of the distal end of the longitudinal cavity may especially be advantageous in case the bone tissue around the bone anchor is not homogeneous and/or not isotropic: the structure of the distal cavity end ensures that also then the material is pressed out through the different exit opening in approximately equal amounts and not for example only through the exit opening where the resistance is smaller compared to the other exit openings. Especially if the material is thermoplastic material and is positioned in the longitudinal cavity in a solid state, it will have little possibility to evade the hydrostatic pressure by flowing back towards proximally and out of the opening where the resistance is the lowest. This is because the material is predominantly liquefied in contact with the distal end of the cavity and prevented from flowing back therefrom by the remaining thermoplastic material being pressed towards distally during the process. In this way the structures of the distal end serve for directing the material to the respective exit opening.

For example, the structures of the distal end comprise may comprise directing walls that extend radially between the inner portions of the channels.

The bone anchor according to the invention consists of a medically acceptable metal, polymer of ceramic material and it is preferably manufactured using an additive manufacturing method. The bone anchor consists e.g. of a suitable titanium alloy, a CoCr-alloy or a molybdenum-rhenium alloy and is manufactured using a selective laser sintering a process, a selective laser melting process or an electron beam melting process.

The present invention also concerns a set of a fenestrated bone anchor as described in the present text together with a thermoplastic element in a solid state, the thermoplastic element having a shape adapted to be inserted into the longitudinal cavity from its proximal end and capable of being liquefied by being pressed against the distal end of the cavity while energy, especially mechanical vibration energy, is coupled into the thermoplastic element.

Such set may further comprise a sonotrode shaped to couple the mechanical vibration energy into the thermoplastic element. The sonotrode may especially have a distal end adapted to be inserted into the longitudinal cavity from its proximal end so as to liquefy a final portion of the thermoplastic element. In addition or as an alternative to the sonotrode, the set may have a guiding sleeve or guiding tube, the guiding sleeve or guiding tube adapted to be coupled to the bone anchor so as to guide the thermoplastic element upon insertion into the longitudinal cavity and/or within the longitudinal cavity.

An exemplary embodiment of the bone anchor according to the invention which is made of a titanium alloy and is suitable for fixating a lumbar spinal fusion device as disclosed in the above-mentioned publication WO2010/096942 has about the following dimensions: overall length (head and shaft) 25 mm, axial position of the lateral channels 3 to 4 mm below the plate, axial length, distal width and proximal width of cross section of lateral channels 3.8 mm, 1.8 mm, 1.4 mm, wall thickness of shaft below head and in the area of the lateral channels 0.7 mm and 0.6 mm.

The fenestrated anchor according to the invention may further be part of a surgical system comprising the anchor and an implant with a through opening adapted to the anchor. Such a system may have the following features.

The surgical system comprises an implant with at least one through opening defining an opening axis and at least one bone anchor with a head and a shaft and an anchor axis, the through opening and the bone anchor being adapted to each other for the anchor shaft to be able to pass through the through opening and the anchor head to be retained by a proximal surface of the implant or in the through opening in a final position. For locking the anchor in said final position, the system further comprises at least one locking element being moveable between a relaxed position in which it protrudes from a general level of a surface portion of the anchor or the through opening and a resiliently tensioned position in which it protrudes less from or is substantially flush with said level. Therein the locking element is an integral part of the bone anchor or of the implant, constituting a part of the named surface portion and of a bulk of the anchor or the implant situated underneath this surface portion. A void delimits the locking element in the surface portion and further extends underneath the locking element or through the named bulk.

The locking element of the system cooperates in a per se known manner, if arranged on the bone anchor, with corresponding surface portions on the implant, preferably within the through opening, and, if arranged in the through opening, it cooperates with corresponding surface portions of the anchor, preferably of the anchor head. Such locking surfaces are in particular inner surfaces of grooves or depressions or portions of a distal surface of the implant or of a proximal surface of the anchor. For guiding the movement and tensioning of the locking element on moving the bone anchor in a distal direction in the through opening, the locking element comprises in a per se known manner a ramp leading parallel to the direction of this movement from a lower to a higher level, wherein, if the locking element is arranged on the bone anchor, the higher level is situated proximally of the lower level, and, if the locking element is arranged in the through opening, the higher level is arranged distally of the lower level. The locking element and the locking surfaces are designed in a per se known manner for a loose fit in the locked position.

The locking element of the system is constituted as a resilient beam being supported on one beam end (resiliently pivoting cantilever) or on two opposite beam ends (resiliently bending beam), wherein the resilient beam is cut out of the bulk of the bone anchor or the implant, i.e. it is delimited by a void extending at least along a beam length and, in the case of a sufficiently thin bulk portion, extends right through this bulk portion (beam thickness substantially the same as local bulk thickness), or, in the case of a thicker bulk portion, extends additionally underneath the beam (beam thickness constituting part of local bulk thickness).

In a relaxed position, the resilient locking element protrudes from a general level of a surface portion of the bone anchor or the through opening in which it is situated. On moving the bone anchor into its final position relative to the through opening, the resilient locking element is pivoted or bent out of the relaxed position to a resiliently tensioned position in which it does not protrude or protrudes less from the general level of the surface of the anchor or the through opening, and, when the final position is reached, it snaps back into its relaxed position.

The implant and/or the bone anchor of the system according to the invention are both made of a medically acceptable metal, polymer or ceramic material, wherein particularly the one of the bone anchor and the implant (or the part thereof comprising the through opening) which comprises the locking element is preferably manufactured using an additive manufacturing process (3D printing process). Bone anchor and/or implant (or part thereof) consist e.g. of a suitable titanium alloy, a CoCr-alloy or a molybdenum-rhenium alloy and are manufactured using a selective laser sintering a process, a selective laser melting process or an electron beam melting process.

One of a plurality of preferred embodiments of the bone anchor according to the invention is part of a system constituting an interbody fusion device of the stand-alone type comprising an interbody fusion implant, a bone plate with a plurality of through openings and a plurality of bone anchors adapted to the through openings of the plate and suitable for fixating the plate relative to two neighboring vertebral bodies. Known such systems are e.g. disclosed in the publication WO 2010/096942. The plate of the system may constitute a fully separate element, may be fixed on the interbody implant or may form one integral part therewith. The interbody fusion implant is positioned between two neighboring vertebral bodies, the plate is arranged on the anterior or on a lateral side of the two vertebral bodies and is fixated thereto with the aid of the bone anchors. The bone anchors preferably used in the system are elongate fenestrated bone anchors with a head and a shaft, which bone anchors are driven into the bone tissue of the vertebral body through the through openings of the plate, and are anchored in the vertebral body e.g. with the aid of a material having thermoplastic properties and being liquefied by application of e.g. vibration energy within a central cavity of the fenestrated anchor and pressed to the outside of the anchor through lateral channels to re-solidify preferably within the trabecular structure of the bone tissue surrounding the anchor or between the bone tissue and the anchor.

The implant belonging to the system of bone anchor and bone implant may have the following features.

The bone implant comprising the system according to invention is a load-bearing bone implant suitable for being implanted in a human or animal patient between surfaces of live bones or bone fragments, suitable for bearing at least temporarily loads acting between these bones or bone fragments, and suitable for being integrated between the bones or bone fragments by bone growth after surgery. Such bone implants are applicable in surgical procedures such as e.g. spinal fusion, arthrodesis and osteotomy.

The named bone implant comprises a porous implant body with an open porosity constituting throughout the porous implant body a three-dimensional network of porosity channels of dimensions suitable for bone ingrowth, which porous implant body constitutes two substantially opposite body surfaces (ingrowth surfaces) to be positioned against the live bone tissue on implantation. In addition to the porosity channels, the porous implant body comprises a plurality of supply channels, wherein each one of the supply channels has a mouth in at least one of the ingrowth surfaces and extends into or preferably through the porous implant body substantially parallel to the force lines of the force field acting on the implant in the implanted state. Therein the supply channels have cross sections larger than the cross sections of the porosity channels, but small enough for being bridgeable by spontaneous bone growth without bone growth enhancing material positioned in the channels.

The porosity channels of the porous implant body of the bone implant have cross sections with diameters in the range of 0.1 to 0.7 mm diameter, preferably in the range of 0.4 to 0.6 mm. The supply channels have cross sections of any suitable form of which the smallest dimension is in the range of 1 to 3 mm and, throughout the porous implant, they have distances from each other in the range of 2 to 6 mm, preferably in the range of 3 to 5 mm. The total volume of the porosity channels and the supply channels together constitute 40 - 80% of the volume of the porous implant body, preferably 60 - 80%).

The design of the porous implant body of the bone implant is based on the following findings. Bone ingrowth into porous implant body structures more or less mimicking the trabecular structure of natural bone tissue is successful only to a depth of 1 to about 3 mm (probably limited by the supply of the ingrown bone tissue by diffusion only). Spontaneous bone growth, i.e. bone growth within weeks after surgery, will bridge gaps of a width of not more than about 3 mm. In larger cavities (all dimensions larger than about 3 mm), bone growth will primarily cover the walls of the cavity, wherein in such cavities having a smallest dimension of not more than about 10 mm (7 to 10 mm), long term bone growth, i.e. bone growth within months, will be able to bridge the cavity, and wherein even larger cavities (all dimensions larger than 7 to 9 mm, critical size defect) do not fill with bone tissue at all, unless they are filled with a bone growth enhancing material such as e.g. bone graft material. No or hardly any ingrowth of bone tissue into the implant is found from implant surfaces other than the implant surfaces (ingrowth surfaces) being, in the implanted state, in direct contact with live bone tissue of the patient, i.e. ingrowth of bone tissue into the implant is highly anisotropic.

Based on the above listed findings, the supply channels of the porous implant body of the bone implant to the invention originate from the ingrowth surfaces, have cross sections large enough for allowing ingrowth of bone tissue including supply means such as in particular vasculature and lymph channels and small enough for being bridged, i.e. completely filled with bone tissue, and, if suitably distanced from each other (and possibly from ingrowth surfaces) throughout the porous implant body enable bone growth in all location thereof. Furthermore, it is found that supply channels longer than about 20 mm are preferably equipped with an enlarged mouth portion.

The orientation of the supply channels along the force lines is advantageous from a mechanical point of view. In most considered cases, these force lines extend substantially parallel to each other from one of the ingrowth surfaces to the opposite other one. Therefore, in a preferred arrangement, the supply channels extend substantially parallel to each other from one ingrowth surface towards the other one, wherein, for being able to supply the largest porous volume with the smallest number of supply channels, the latter are arranged in a hexagonal system, wherein, in a cross section through the channel arrangement, each channel has six neighboring channels at a same distance.

The bone implant comprises, in addition to the porous implant body, a support frame which partially surrounds and possibly also penetrates the porous implant body. The support frame is made of a suitable, preferably non-porous material. The support frame constitutes at least part of a proximal implant surface (trailing surface on implantation, usually not an ingrowth surface) and members e.g. running along edge regions of the porous implant body, which edge members e.g. surround ingrowth surfaces without protruding from the latter, extend in ingrowth surfaces, or penetrate the porous implant body. Outer surfaces of the support frame may be equipped with a per se known osseointegration enhancing surface structure or coating, at least where they are to be positioned against surfaces of live bone tissue and where osseointegration is desired.

The bone implant may comprise further elements of a substantially non-porous material. Such further elements are e.g. surface elements, which are arranged in the ingrowth surfaces and flush therewith and may serve for facilitating implantation by reducing friction between the ingrowth surface and the surface of the live bone tissue without impairing the direct contact between the porous implant body and the live bone tissue. The surface elements e.g. surround mouths of supply channels and therewith help to prevent mechanical damage of the mouth edges. Further such elements may also be struts extending along the wall of selected ones of the supply channels and serving as stiffening and supporting means.

While the dimensions of the members of the support frame depend on the type and size of the implant and on the load the latter has to bear when implanted, the surface elements have dimensions which are as small as possible. In a medium interbody fusion implant, the members of the support frame have e.g. widths and depth (into the porous implant body) in the range of a few millimeters (2 to 5 mm) and the surface elements have widths and depths of about one millimeter (0.8 to 1.5 mm). The same applies to the struts.

The bone implant may, in the same manner as known such implants, further comprise a larger cavity to be filled with bone growth enhancing material, wherein this cavity may, in a per se known manner, reach from one ingrowth surface to the other one. Furthermore, the bone implant may comprise per se known mechanical retention means.

The porous implant body is made of a suitable, medically approved metal, polymer or ceramic material, e.g. titanium (grade 1-4), titanium alloy (e.g. Ti-6Al-4V or Ti-7Al-11Nb), resorbable magnesium alloys, PEEK, polylactide (resorbable), biocomposites, zirconium oxide, aluminum oxide or mixtures of the two oxides, or calcium phosphate (tri-calciumphosphate, hydroxyapatite, both resorbable). The porous implant body is preferably manufactured using an additive manufacturing method, e.g. selective laser sintering, e-beam melting, or fused deposition (in particular for polymer and ceramic material). For strengthening the porous structures manufactured e.g. with the named 3D-printing methods they may be compacted by HIP-processes. For rendering them more bio-active, they may be surface treated in galvanic or non-galvanic processes (e.g. etching or nano-deposition).

Preferably, the support frame and possibly also further elements and retention means of the bone implant are made of the same material as the porous implant body and are manufactured in the same additive process as the porous implant body such forming one integral part with the latter.

The bone implant is particularly suitable for applications in which the ratio between implant surface and implant volume is relatively small (relatively large implant bulk), in particular it is suitable for applications requiring an implant thickness (distance between opposite ingrowth surfaces) in the range of 4 to about 30 mm. Furthermore, it is particularly suitable for applications in which provision of permanent mechanical retention structures is limited, and for which long-term success of the corresponding surgical procedure is highly dependent on successful bone growth after surgery. This is e.g. the case for interbody fusion devices used in spine surgery, for various implants used in other arthrodesis methods, and for wedge-shaped implants used in various osteotomy procedures.

One of a plurality of preferred embodiments of the bone implant is an interbody fusion implant, which may be of the stand-alone type or of the type which is combined with further instrumentation such as e.g. systems of pedicle screws and spinal rods. Known such implants are e.g. disclosed in the publication WO2010/096942. The devices as disclosed in the named publication comprise an interbody implant and, if of the stand-alone type, a plate which constitutes a separate element or is fixedly arranged on the interbody implant. The plate is fixated on the anterior or on a lateral side of the two vertebral bodies between which the interbody implant is positioned. For the fixation of the plate, in particular fenestrated headed bone anchors are proposed, which bone anchors are driven into the bone tissue of the vertebral body through through openings provided in the plate and are anchored in the vertebral body e.g. with the aid of a material having thermoplastic properties and being liquefied by application of e.g. vibration energy within a longitudinal cavity of the anchor and pressed to the outside of the anchor through lateral channels (fenestration) to re-solidify preferably within the trabecular structure of the bone tissue surrounding the anchor or between the bone tissue and the anchor.

### BRIEF DESCRIPTION OF THE DRAWINGS

The invention is described in further detail in connection with the appended Figs., wherein:
- **Figs. 1A/B/C**: illustrate an exemplary embodiment of the fenestrated bone anchor according to the invention, the embodiment comprising three lateral channels with pear-shaped cross sections;
- Fig. 2: is a plan view of the fenestrated bone anchor according to Figs 1A/B/C (viewing direction towards the head of the anchor)
- Figs. 3 and 4: are cross sections through further exemplary embodiments of the bone anchor according to the invention, the
- **Figs. 5A/B/C**: embodiments comprising an increase in wall thickness in a direction towards the lateral channels; illustrate the principle of an exemplary locking element of the system, the illustrated locking element having the form of a resilient cantilever;
- **Figs.** 6A/B/C: illustrate the principle of an exemplary locking element of the system, the illustrated locking element having the form of a resilient bending beam;
- **Figs.** 7A/B/C: shows an exemplary embodiment of a bone anchor of the system, the bone anchor comprising a locking element in the form of a resilient cantilever, the cantilever length extending substantially parallel to the movement of the bone anchor relative to the through opening;
- **Figs. 8A/B**: show an exemplary embodiment of a bone anchor of the system, the bone anchor comprising a locking element in the form of a resilient cantilever, the cantilever length extending substantially perpendicular to the movement of the bone anchor relative to the through opening;
- Figs. 9A/B/C: show an exemplary embodiment of a bone anchor of the system, the bone anchor comprising a locking element in the form of a resilient bending beam, the beam length extending substantially perpendicular to the movement of the bone anchor relative to the through opening;
- **Figs. 10 and**: 11illustrate the principle of a bone implant, the bone implant being shown in section perpendicular to ingrowth surfaces of the porous implant body;
- Figs. 12, 13, 14: are cross sections of exemplary embodiments of supply channel arrangements;
- Fig. 15: illustrates a further exemplary embodiment of the bone implant;
- Fig. 16: shows an interbody fusion device of the stand-alone type comprising a plate with through openings suitable for being fixated to vertebral bodies with the ais of bone anchors.

DESCRIPTION OF THE PREFERRED EMBODIMENTS The present invention is defined in claim 1 while preferred embodiments are set forth in the dependent claims.

Associated manufacturing methods are also described herein to aid understanding the invention. These methods do not form part of the claimed invention.

In the whole of the present text the term "proximal" is used to designate a position nearer a surgeon and the term "distal" a position nearer the patient. Similarly, a proximal direction means a direction against the surgeon and a distal direction a direction against the patient or further into the patient. Each item has a proximal end and a distal end, the distal end being the leading end on implantation and the proximal end being the trailing end on implantation. Most described items have a proximal end and a distal end and an axis extending therebetween wherein this axis on implantation coincides or is parallel to the implantation direction and wherein the length of this axis may or may not be the longest extension of the item.

In the appended figures, similar elements or elements with same functions are designated with same reference numerals.

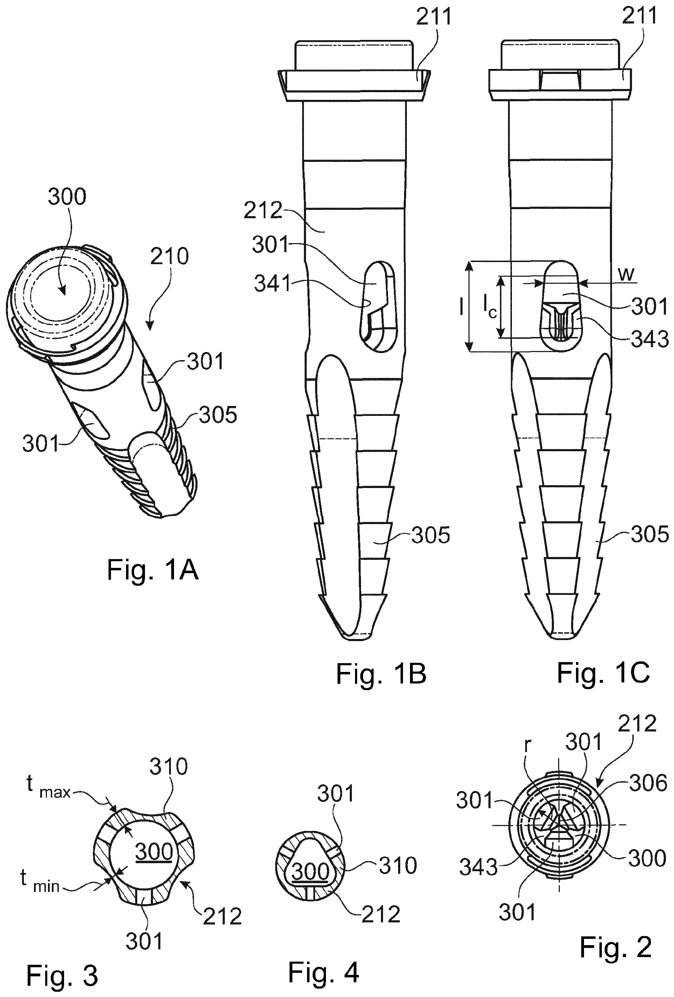

**Figs 1A****/B/C** illustrate an exemplary embodiment of the fenestrated bone anchor according to the invention, wherein Fig. 1A is a three-dimensional illustration of the anchor, and Figs. 1A and 1B are lateral views differing from each other by an angle of 90° between the corresponding viewing directions. The bone anchor 210 comprises a head 211 and a shaft 212. The longitudinal cavity 300 reaches through the head 211 into the shaft to a closed distal end and it is connected with the circumferential surface of the shaft by three lateral channels 301. As described further above, the lateral channels 301 have a pear-shaped cross section, i.e. an axial length l (see Fig.1C) greater than a circumferential width w. The width w decreases in a proximal direction. The cross section has in a per se known manner no sharp corners such preventing load concentrations. The lateral channels 301 have outer mouths positioned all at the same axial position situated in the proximal half of the shaft axis, the closed distal end of the longitudinal cavity 300 is situated about half way between the proximal shaft end and the distal shaft end. The circumferential surface of the distal part of the shaft is equipped with circumferential ribs constituting exemplary means for retaining the anchor in a bone opening provided for its implantation.

The overall pear shape of the lateral channels is such that the sidewalls 341 and the edges of their mouth are not parallel to each other also in a middle region (between the dotted lines in Fig. 1C), the middle region having a substantial axial extension l_{c} compared to the axial extension l of the axial channel. Also, the average circumferential width w of a proximal half of the lateral channel (above the dashed line in Fig. 1C) is substantially smaller than the average circumferential width of the distal half. An angle between the sidewalls and the longitudinal axis in the depicted embodiment is about 5°

**Fig. 2** is a plan view of the fenestrated bone anchor as shown in Figs. 1A/B/C (viewing direction from the head towards the tip of the anchor). It illustrates the distal end of the longitudinal cavity, which is equipped with relatively sharp edges 306 or peaks serving as energy concentrators during the process of liquefaction of a material having thermoplastic properties being positioned in the longitudinal cavity and being pressed against the distal end of the longitudinal cavity, while ultrasonic vibration energy is applied to it. Also visible in Fig. 2 are the inner mouths of the lateral channels 301. Further exemplary embodiments of distal cavity ends suitable for the fenestrated bone anchor according to the invention are e.g. disclosed in the publication WO 2011/054122.

More in concrete, the closed distal end of the longitudinal cavity 300 has a shape different from circularly symmetrical about the axis. Rather, the channels 301 have an inner portion at radial positions within a radius of the longitudinal cavity 300 in addition to an outer portion formed by the (pear-shaped) opening (exit opening) in the wall around the cavity 300. Thus, the distal end of the longitudinal cavity is angularly structured so as to direct different portions of the material (material having thermoplastic properties or bone cement) to the different exit openings.

In the embodiment shown in Figs. 1A-2, the structures of the distal end comprise directing walls 343 that extend radially between the inner portions of the channels 301. Proximal edges of such walls may optionally in addition to serving as directing structures serve as energy directors in the above-mentioned sense.

**Figs. 3 and 4** are cross sections through the shaft 212 of further exemplary embodiments of the fenestrated bone anchor according to the invention. The section plane of these cross sections is situated in the axial position of the lateral channels 301 and illustrate in particular the wall 310 between the longitudinal cavity 300 and the circumferential shaft surface. This wall has a wall thickness which increases in a circumferential direction towards each lateral channel 301 from a minimum wall thickness tₘᵢₙ in locations between the lateral channels 301 and a maximum wall thickness tₘₐₓ where the wall meets the lateral channel 301. The increase of the wall thickness is e.g. in the range of 20%. This design measure renders the cross section of the anchor shaft 212 and the cross section of the longitudinal cavity 300 to be different, wherein in the illustrated case showing three lateral channels 301 the one cross section is circular and the other one is three-lobed having a circular envelope. Accordingly, for an anchor having two lateral channels, the lobed cross section is like an oval (two-lobed), for four channels it is four-lobed, and so on.

The embodiment of wall 310 according to Fig. 3 for which the cross section of the shaft is lobed and the cross section of the longitudinal cavity is circular is advantageous when using the corresponding anchor for the above briefly described anchoring process with the aid of an element of a material having thermoplastic properties, as this element may be a simple pin having a circular cross section. On the other hand, it either necessitates a bone opening provided for the anchor having the trilobal cross section of the anchor shaft or a larger opening having a circular cross section substantially corresponding to the circular envelope or the trilobal shaft cross section. In an axial direction, in particular in a proximal direction, away from the lateral channels, the lobed cross section preferably changes gradually into a circular cross section such guaranteeing good guidance of the anchor in an also circular through opening of e.g. a bone plate. The embodiment of the wall 310 according to Fig. 4 is advantageous for an anchor of an overall circular cross section, e.g. a fenestrated screw, but for the above-mentioned anchoring process it necessitates an element of the material having thermoplastic properties in the form a pin with a non-circular cross section.

Further embodiments of the fenestrated bone anchor according to the invention differ from the embodiments illustrated in the appended figures by not comprising a head or a head of a different form, by comprising instead of three lateral channels only one or e.g. 2 or 4 lateral channels, by the lateral channels not being situated further proximal but in the middle of the axial shaft length or further distal, by the longitudinal cavity reaching right through the shaft and having an open distal end, by not having a general circular shaft cross section but a shaft cross sections as e.g. listed in the introductory part of the present disclosure, by the lateral channels being situated not in a same axial position but in differing axial positions, and/or by comprising different or no retention means as e.g. listed in the introductory part of the present disclosure. All the named alternative features can be selectively used or combined for a plurality of further exemplary embodiments of the fenestrated bone anchor according to the invention and designed for specific applications.

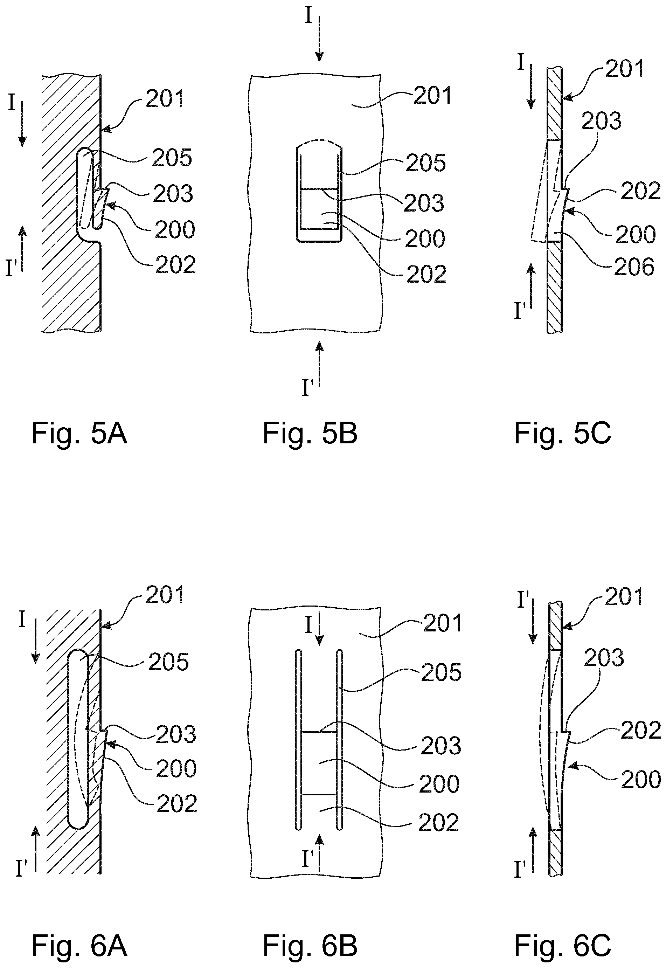

**Figs. 5A****/B/C and 6A/B/C** illustrate each in a schematic manner the principle of exemplary embodiments of locking elements 200 suitable for a any embodiment of the fenestrated bone anchor according to the invention, wherein the bone anchor cooperates with a through opening provided in an implant, bone anchor and implant forming together a system. Each one of these Figs. shows a surface portion 201 of a bone anchor or through opening of an implant belonging to the system, wherein the locking element 200 is arranged within this surface portion 201. Figs. 5A, 5C, 6A and 6C are sections through the surface portion 201 and the locking element 200, the section plane being oriented substantially parallel to the axis of the bone anchor or the through opening, or to the implantation direction respectively. These Figs. show the locking element 200 in its relaxed position (uninterrupted lines), in which it protrudes from the general level of the surface portion 201, and in its resiliently tensioned position (interrupted lines) in which it protrudes less or not at all from the general level of the surface portion 201 (flush or countersunk). Figs. 5B and 6B are plan views of surface portion 201 and locking element 200. The surface portions 201 are preferably portions of the circumferential surface of either the bone anchor head or the bone anchor shaft and are substantially convex. However, the illustrated surface portions 201 may also be portions of the inside surface of the through opening of the implant and, in such a case, are substantially concave. Locking elements arranged on the bone anchor (shaft or head) are moved relative to the through opening in a direction illustrated with an arrow I (implantation direction). Locking elements arranged in the through opening are moved relative to the bone anchor in an opposite direction indicated with the arrow I'.

The locking element 200 comprises a protrusion with a guiding ramp 202 and a locking surface 203, wherein the guiding ramp 202 is situated upstream or downstream of the locking surface, depending on the moving direction I or I'. The illustrated locking elements comprise protrusions with ramps 202 and locking surfaces 203 extending over the full width of the locking element. Alternatively, the protrusion may be narrower.

The locking elements illustrated in **Figs. 5A****/B/C** have the form of a resilient cantilever pivoting in a plane substantially parallel to the directions I and I' or to the axis of the bone anchor or the through opening respectively. The void 205 delimiting the cantilever extends along its length and on one side along its width also, and, depending on the thickness of the bulk beneath the surface portion 201 and on the bulk material, extends furthermore underneath the locking element (Fig. 5A) or fully through the bulk underneath the locking element (Fig. 5C).

The locking elements illustrated in **Figs 6A****/B/C** have the form of a resilient bending beam with a bending movement in a plane substantially parallel to the directions I and I' or to the axis of the bone anchor or the through opening respectively. The void 205 delimiting the bending beam extends along its length limiting its width, and, depending on the thickness of the bulk beneath the surface portion 201 and on the bulk material, extends furthermore underneath the locking element (Fig. 6C) or fully through the bulk underneath the locking element (Fig. 6C).

Dimensions of the cantilever or bending beam of the locking elements need to be adapted to the bulk material and bulk thickness in the region in which the locking element is situated. Generally speaking, deformation of the cantilever or bending beam needs to remain in the elastic range (less than 1%). For locking elements as shown in Figs. 5A and 6A, there is more design freedom regarding thickness and therewith length of the locking element than there is for the locking elements as shown in Figs. 5C and 6C, for which the thickness of the locking element is given by the bulk thickness. Whereas the embodiments of Figs. 5A and 6A only require a minimum bulk thickness, the embodiments according to Figs. 5C and 6C can only be realized within a limited range of bulk thickness (substantially limited to cannulated bone anchor). Applicability of the embodiments according to Figs. 5C and 6C is further limited by the fact that these locking elements constitute through openings through e.g. a wall of a cannulated bone anchor which, in the location of the locking element may not be tolerable, in particular when the anchor is used e.g. for an augmentation process with the aid of a flowable material such as a bone cement.

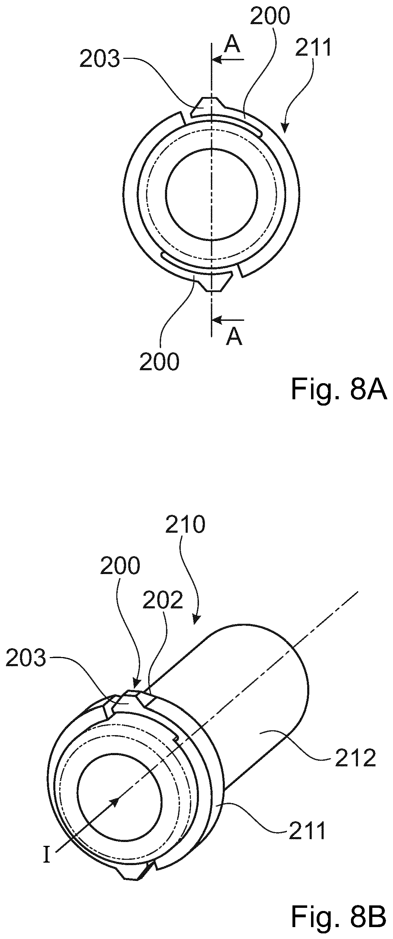

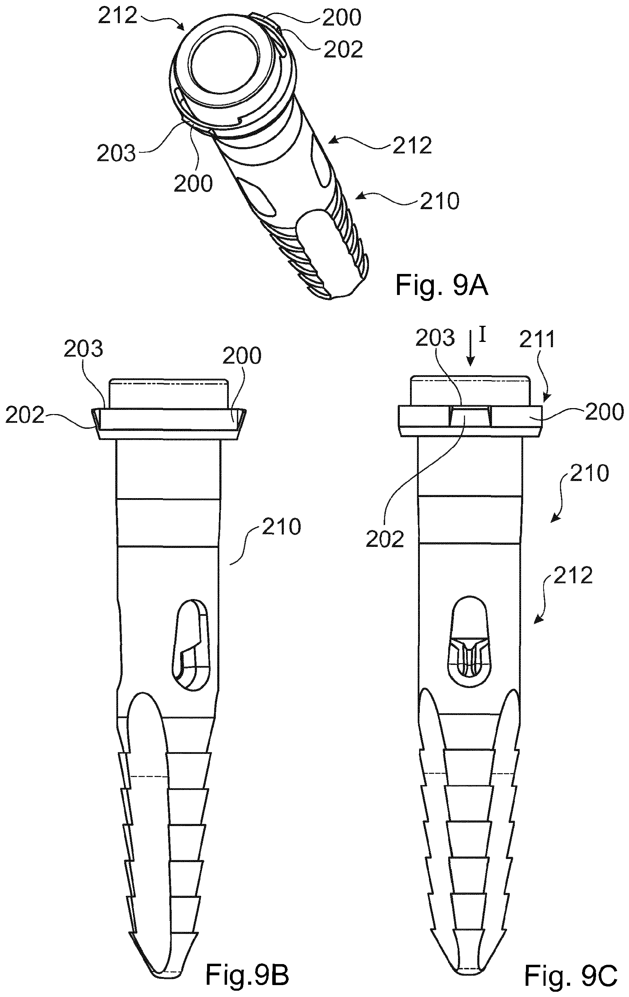

The locking elements as illustrated in Figs. 5A/B/C and 6A/B/C comprise cantilevers or bending beams with a length extending substantially parallel to the directions I and I' or to the axis of the bone anchor or the through opening on which they are arranged. This is not a necessary feature of the locking element of the system according to the invention. The lengths of such a cantilever or bending beam can also extend at an angle to the directions I and I' or substantially perpendicular to them, wherein ramp and locking surface still have to be arranged following each other in the direction of the movement of bone anchor and through opening relative to each other. This means for a cantilever or bending beam length extending substantially perpendicular to the direction of this movement, that ramp and locking surface are arranged beside each other over the width of the cantilever or bending beam width. Two exemplary embodiments of locking elements in the forms of cantilever and bending beam having a length extending substantially perpendicular to the named moving direction or anchor axis respectively are illustrated in Figs. 8A/B and 9A/B/C.

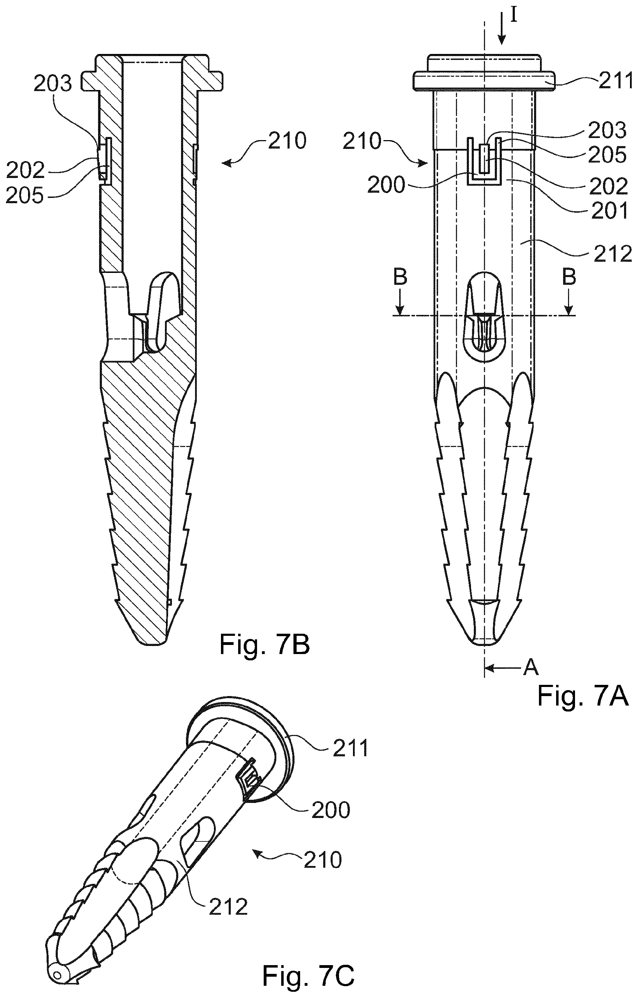

**Figs. 7A****/B/C** illustrate an exemplary embodiment of a bone anchor of the system according to the invention. The bone anchor is shown viewed from a lateral side (Fig. 7A), sectioned along its axis (Fig. 7B), and in a three-dimensional representation (Fig. 7C). The bone anchor 210 comprises a head 211 and a shaft 212 on which two opposite integrated locking elements 200 of the type being illustrated in Figs. 5A and 5B are provided. Each one of the locking elements 200 has the form of a resilient cantilever surrounded on three sides by a void 205 and having a length extending parallel to the anchor axis and the direction I. The locking element further comprises a ramp 202 and a locking surface 203 which, as best seen from Fig. 7A, have a width smaller than the width of the cantilever, and which, as seen best from Fig. 7B, are arranged adjacent to each over the cantilever length, wherein, in the direction I, the ramp 202 is arranged downstream of the locking surface 203.

**Figs. 8A****/B** show a further exemplary embodiment of the bone anchor 210 of the system according to the invention. The bone anchor 210 is shown in a three-dimensional representation (Fig. 8A) and in a plan view viewed against its proximal surface (Fig. 8B). The bone anchor 210 comprises a head 211, a shaft 212 (only partially shown in Fig. 8B), and arranged on the head, two locking elements 200 in the form of cantilevers as illustrated and described in connection with Figs. 8A and 8B. Other than shown in Figs. 8A and 8B, the cantilever length is not oriented parallel to the direction I or the anchor axis respectively, but it is oriented perpendicularly to the latter, i.e. the cantilever length extends circumferentially and the ramp 202 and the locking surface 203 are arranged adjacent to each other over the cantilever width, with the ramp 202, relative to the direction I being arranged downstream of the locking surface 203.

**Figs. 9A****/B/C** show a further exemplary embodiment of the bone anchor 210 of the system according to the invention. The bone anchor is shown in a three-dimensional representation (Fig. 9A) and in two lateral views (Figs. 9B and 9C, angle between the two viewing directions of 90°). The bone anchor 210 comprises again a head 211 and a proximally cannulated shaft 211 and, arranged on the head 211, two opposite locking elements 200 in the form of bending beams as illustrated in Figs. 6A and 6B. Other than in Figs. 6A and 6B, the beam length does not extend substantially parallel to the anchor axis but substantially perpendicular to it, i.e. circumferentially. extending substantially perpendicular to the anchor axis or the direction I respectively. Therefore, the ramp 202 and the locking face 203 of the locking elements 200 are arranged adjacent to each other over the beam width as above further described in connection with Figs. 8A/B.

In all previous Figs., the bone anchor and therewith also the through opening of the implant have substantially circular cross sections. Of course, these cross sections may have other forms, such as e.g. oval, rectangular, polygonal with sharp or blunt edges. This means that the surface portions in which the locking elements are provided are not necessarily curved surfaces.

All bone anchors shown in Figs. 7 to 9 are fenestrated bone anchors comprising a longitudinal cavity and lateral channels and are suitable for being retained in the bone tissue by being augmented using a bone cement or a material having thermoplastic properties. This is not a necessary feature of the system according to the invention. The bone anchor of this system may comprise any per se known retention means, i.e. the bone anchor can be a bone screw (solid, cannulated or fenestrated) or it can comprise retention means in the form of ribs, edges, resilient elements etc. The bone anchor of the system according to the invention may also be a simple headed pin with a possibly rough circumferential surface and being suitable to be retained in the bone tissue by a press fit.

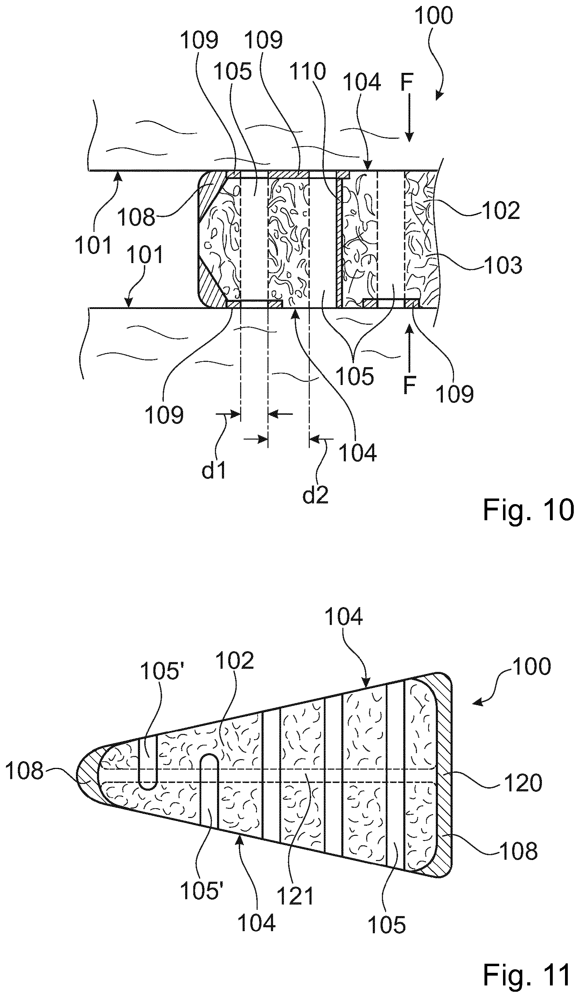

**Figs. 10 and 11** illustrate the principle of the structure of the porous implant body of the bone implant, wherein Fig. 10 illustrates an implant example with two opposite substantially parallel ingrowth surfaces, e.g. an interbody fusion implant, and Fig. 11 illustrates an implant example with two ingrowth surfaces extending at an angle relative to each other, e.g. a wedge-shaped implant as often used in osteotomy procedures.

The implant 100 shown in **Fig. 10** in section perpendicular to the live bone surfaces 101 between which it is implanted is e.g. an interbody fusion implant, the live bone surfaces 101, in such a case, being suitably prepared lower and upper surfaces of neighboring vertebral bodies, wherein forces acting on the implanted implant are mainly compressing forces (arrows F) acting substantially perpendicular to the live bone surfaces 101 or the ingrowth surfaces 104 respectively. The implant 100 comprises a porous implant body 102 of an open porosity constituting a three-dimensional network of porosity channels 103. Ingrowth surfaces 104 are opposite surfaces of the porous implant body 102 which, in the implanted state of the implant, are in direct contact with the live bone surfaces 101. The implant body further comprises a plurality of supply channels 105 extending between the two ingrowth surfaces 104 substantially in the direction of the forces acting on the implanted implant. Whereas the porosity channels103 have cross sections with diameters in the range of a few tenths of a millimeter, the supply channels 105 have cross sections of a diameter d1 of a few millimeters (preferably 1 - 3 mm). The distances d2 between the supply channels 105 are in the range of 2 to 6 mm (preferably 3 to 5 mm). Preferably, but not necessarily, the distances d2 between the supply channels 105 are, throughout the porous implant body, about constant, and, in no case, are larger than about 5 to 6 mm. This means that the supply channels 105 preferably extend through the porous implant body 100 in a substantially regular pattern.

Also shown in Fig. 10 are substantially non-porous edge members 108 of a support frame surrounding at least partially the porous implant body 102, as well as substantially non-porous surface elements 109 arranged to extend flush in the ingrowth surfaces 104 and e.g. surrounding mouths of supply channels 105 and/or extending between such mouths. Also shown in Fig. 11 is a strut 110 extending along the wall of one of the supply channels 105, wherein more than one strut 110 may be provided in one and the same supply channel 105 and wherein distances between struts are to be in the range of 0.5 to 2 mm. Surface elements 109 and struts 110 may be provided for each one of the supply channels 105 or for selected ones only.

**Fig. 11** is a very schematic representation (again in a section perpendicular to the ingrowth surfaces 104) of an exemplary wedge-shaped implant 100, which implant is possibly suitable for use in an osteotomy operation. The main features of the implant are the same as the features of the implant as shown in Fig. 10, namely the porous implant body 102 with ingrowth surfaces 104 (in Fig. 11 two ingrowth surfaces at an angle relative to each other), supply channels 105 extending from one ingrowth surface 104 towards the other one, and members 108 of a support frame. In addition to the supply channels 105 extending from one ingrowth surface 104 to the other one and having a mouth in either one of the ingrowth surfaces 104, there are illustrated two blind supply channels 105', which only have one mouth and a closed end opposite the mouth. For guaranteeing satisfactory supply for bone ingrowth beyond the closed end, the dead end is to be positioned not more than 5 to 6 mm (preferably between 3 to 5 mm) distanced from the neighboring ingrowth surface (same as distance between supply channels). The support frame of the implant according to Fig. 11 differs from the support frame of the implant according to Fig. 10 in that it comprises, in addition to an edge member 108 arranged at the distal end of the porous implant body 102, a member 120 constituting a proximal implant surface and at least one central member 121 extending through the implant body. The implant according to Fig. 11 may or may not comprise surface elements or struts (none shown) as described further above in connection with Fig. 10.

Any embodiment of the bone implant may comprise in addition or alternatively to supply channels extending from one ingrowth surface to another one (as shown in Fig. 10), blind supply channels as illustrated in Fig. 11.

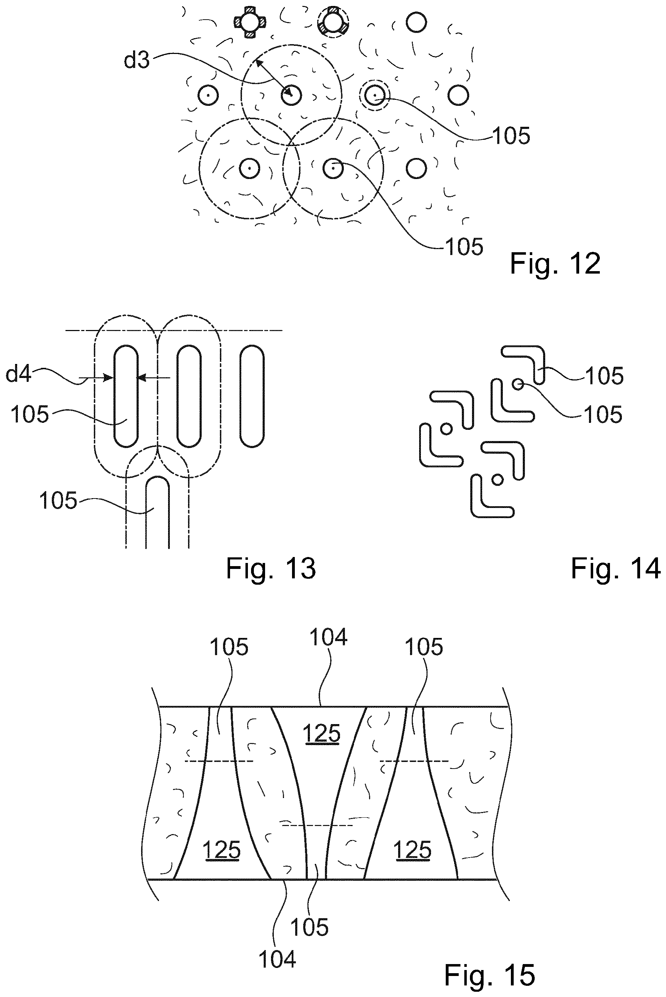

**Fig. 12** further illustrates a preferred embodiment of the arrangement of supply channels 105 in a bone implant, namely the above-mentioned hexagonal arrangement. The arrangement is shown in section substantially perpendicular to the supply channels. In this arrangement, every supply channel 105 has six nearest neighbor channels, wherein all positions between the channels can be safely supplied if the distance d3 (radius of circles marked with dash-dotted lines) corresponds to the given limit of 1 to 3 mm. With the hexagonal arrangement the largest volume of porous structure can be supplied with the smallest number of supply channels.

Also shown in Fig. 12 are exemplary supply channels bring equipped with surface elements 109 surrounding supply channel mouths or connecting them and supply channels comprising varying numbers of struts 110, wherein supply channels 105 in any supply channel arrangement and of any bone implant as above described may or may not be correspondingly equipped.

In all previous Figs. the supply channels have a substantially circular cross section. This is not an obligatory feature of the bone implant. On the contrary, in all embodiments of the bone implant, the supply channels may have cross sections of any other form, as long as for these cross sections the smallest dimension is in the range of 1 to 3 mm. This means that the supply channels may have e.g. square, rectangular, slot-shaped, triangular, oval, lobed, pentagonal, hexagonal etc. cross sections, wherein in one implant all supply channels may have the same or different cross sections.

**Figs. 13 and 14** illustrate schematically two supply channel arrangements (sectioned again perpendicular to the supply channels), comprising supply channels having elongate, i.e. slot-shaped cross sections.

The arrangement shown in **Fig. 13** is a staggered arrangement of supply channels 105 with slot-shaped cross sections having a width (smallest dimensions) in the range of 1 to 3 mm, and distances from each other in the given range of 2 to 6 mm, preferably 3 to 5 mm. Dash-dotted lines indicate, as in Fig. 12, the volume of porous structure which can be supplied by each one of the supply channels 105.

The arrangement shown in **Fig. 14** comprises supply channels 105 with slot-shaped and supply channels 105 with circular (or any other shape having a rotational symmetry) cross sections arranged in a regular pattern, and therewith constitutes an example of a supply channel arrangement comprising supply channels with differing cross sections. In any bone implant as above described, supply channels of other and possibly differing cross section shapes may be combined in varying numbers, wherein it is not necessary that the arrangement is as regular as shown in Figs. 12 to 14.

**Fig. 15** illustrates in a very schematic manner an exemplary bone implant, wherein for this implant, the distance between the two ingrowth surfaces 104 is in a range (20 to 40 mm) for which it is preferable to design the supply channels 105 to have enlarged mouth portions 125. Such enlarged mouth portions preferably have dimensions in a range of 3 to 10 mm such that they are still able to be bridged by long-term bone growth without the necessity of bone growth enhancing material. Larger such enlarged mouth portions constituting critical size defects are not recommended because introduction of bone growth enhancing material is not really feasible. As shown in Fig. 15, the enlarged mouth portions 125 of the supply channels 105 are arranged alternatively in one and the other one of the ingrowth surfaces, which makes it possible to guarantee the given distances between the supply channels in the range of preferably 3 to 5 mm. Preferably the axial length of the enlarged mouth portions 125 of the supply channels 105 have a short axial length only, such maximizing spontaneous bone growth filling as much of the supply channels as possible.

All the above described features of bone implants, in particular regarding support frames and cross section shapes and supply channel arrangements are applicable also for embodiments comprising supply channels with enlarged mouth portions.

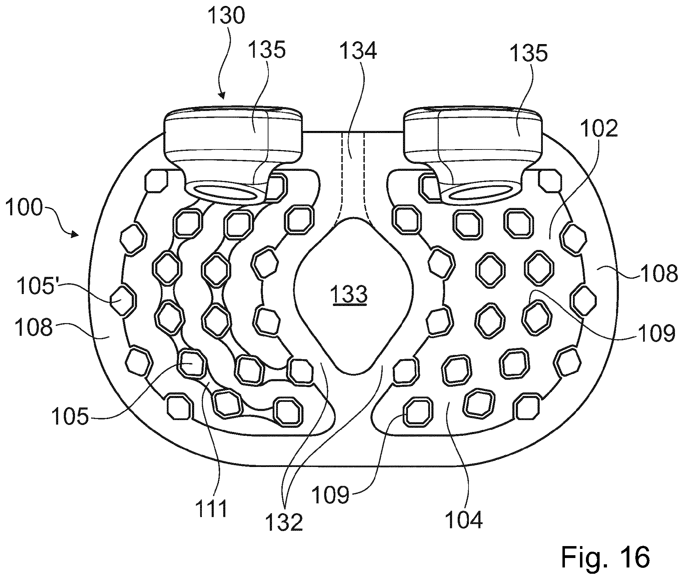

**Fig. 16** is a top view (viewed against one of the ingrowth surfaces 104) of a further exemplary embodiment of the bone implant. The implant 100 is an interbody fusion device of the stand-alone type and comprises, as described above, a porous implant body 102 with supply channels 105 and a support frame with members 108 to be positioned between adjacent vertebral bodies. The interbody fusion device further comprises a bone plate 130 (retention means) and a plurality of bone anchors (not shown) suitable for fixating the bone plate 130 to lateral or posterior wall portions of the vertebral bodies. In the shown embodiment, the bone plate 130 is reduced to a plurality of lobes 135 each comprising a through opening for receiving one of the bone anchors. The porous implant body 102, the support frame members 108, the bone plate 130, and, if applicable, surface elements 109 or struts are preferably made of the same material, as one piece, which is preferably manufactured in one single additive manufacturing process.

The porous implant body 102 comprises supply channels 105 as described above, mouths of the supply channels being at least partly surrounded by surface elements 109, possibly being connected to each other by connecting elements 111 (illustrated on left hand side of ingrowth surface). The support frame comprises edge members 108 and two central members 132, which extend through the porous implant body from the one ingrowth surface to the other one and which may or may not encircle a central cavity 133 suitable for being filled with bone growth enhancing graft material for which purpose this cavity 133 comprises a feed opening 134 connecting it with the proximal surface of the implant.

## Claims

1. A fenestrated bone anchor (210) suitable for being anchored in live bone tissue of a human or animal patient, wherein the bone anchor (210) comprises a shaft (212) with a proximal end, a distal end, a longitudinal axis and a circumferential surface extending from the proximal end to the distal end, wherein the shaft (212) further comprises a longitudinal cavity (300) extending in the direction of the axis from the proximal end towards the distal end and at least one lateral channel (301) extending through a wall (310) of the shaft (212) from the axial cavity (300) to the circumferential surface, the wall (310) having a wall thickness and the lateral channel (301) having a cross section with an axial length (l) and a width (w) smaller than the axial length, and wherein said width (w) increases in a distal direction and, one of additionally or alternatively, said wall thickness increases gradually in at least selected ones of directions towards the lateral channel, wherein the bone anchor comprises a plurality of lateral channels (301) arranged at a same first distance from the proximal end of the shaft (212)..

2. The bone anchor according to claim 1, wherein all lateral channels (301) of the plurality of the lateral channels (301) are regularly spaced from each other around the circumferential surface.

3. The bone anchor according to claim 1 or 2, wherein the first distance is smaller than a second distance between the lateral channel and the distal end of the shaft (212), and/or wherein the first distance is smaller than about one half of a total axial length of the shaft.

4. The bone anchor according to any one of claims 1 to 3, wherein the wall thickness increases towards each one of the lateral channels (301) and a cross section of the bone anchor (210) or of the longitudinal cavity (300) through the plurality of lateral channels (301) has a lobed form having a lobe associated to each one of the channels

5. The bone anchor according to claim 4, wherein in a proximal direction away from the plurality of lateral channels (301), the lobed form of the cross section of the bone anchor gradually transitions to a circular form.

6. The bone anchor according to any one of claims 1 to 5, further comprising a head (211) and being suitable for fixating a bone plate relative to bone tissue, wherein the bone plate comprises a through opening adapted to the bone anchor.

7. The bone anchor according to any one of claims 1 to 6, further comprising retention structures arranged on the circumferential surface of the shaft, wherein the retention structures comprise at least one of a thread, circumferential ribs, sharp edges, teeth, surface roughness, undercut surface structures and an osseointegration enhancing surface coating.

8. The bone anchor according to any one of claims 1 to 7, wherein at axial positions of the at least one lateral channel (301), the stiffness of the anchor (210) against bending increases gradually towards proximally.

9. The bone anchor according to any one of claims 1 to 8, wherein across a substantial portion the lateral channel (301) the polar second moment of area of the bone anchor (210) gradually increases towards proximally.

10. The bone anchor according to any one of claims 1 to 9, wherein an average circumferential width of a proximal half of the at least one lateral channel is substantially smaller than an average circumferential width of a distal half of the lateral channel.

11. A surgical system comprising an implant (100) with at least one through opening defining an opening axis and at least one fenestrated bone anchor according to any one of claims 1 to 10 and being suitable for fixating the implant (100) relative to bone tissue of a human or animal patient, the bone anchor comprising a head and a shaft and an anchor axis, the through opening and the bone anchor (210) being adapted to each other for the shaft (212) to be able to pass through the through opening and the head (211) to be retained by a proximal surface of the implant (100) or within the through opening in a final position, wherein, for locking the anchor in said final position, the system further comprises at least one locking element (200) being moveable between a relaxed position in which it protrudes from a general level of a surface portion of the anchor (210) or the through opening and a resiliently tensioned position in which it protrudes less or not at all from said level, wherein the locking element (200) is an integral part of the bone anchor (210) or of the implant (100), constituting a part of said surface portion and of a bulk of the anchor or the implant situated underneath said surface portion, wherein a void (205) in the surface portion delimits the locking element (200) and further extends underneath the locking (200) element or through said bulk.

12. The system according to claim 11, wherein the locking element (200) is formed as a resilient cantilever or resilient bending beam having a length and a width, wherein the locking element (200) further comprises a ramp and a locking surface being arranged adjacent to each other, wherein the length of the cantilever or bending beam extends substantially parallel to the anchor axis or to the opening axis and the ramp and the locking surface are arranged adjacent to each other over the length of the cantilever or bending beam, wherein the locking element is arranged on the shaft of the bone anchor, wherein the length of the cantilever or bending beam extends substantially perpendicular to the anchor axis or the opening axis and the ramp and the locking surface are arranged adjacent to each other over the width of the cantilever or bending beam, and wherein the locking element is arranged on the head (211) of the bone anchor (210).

13. The system according to claim 11 or 12, wherein the implant (100) is a load bearing bone implant suitable for being implanted in a human or animal patient between surfaces of live bones or bone fragments, suitable for transmitting forces acting between the bones or bone fragments, and suitable for being integrated between the bones or bone fragments by bone growth after surgery, the bone implant comprising a porous implant body (102) and a support frame, wherein the porous implant body comprises:
opposite ingrowth surfaces (104) to be positioned against surfaces (101) of the bones or bone fragments,
an open porosity constituting throughout the porous implant body (102) a three-dimensional network of porosity channels (103) of dimensions suitable for bone ingrowth, and
a plurality of supply channels (105), wherein each one of the supply channels (105) has a mouth in at least one of the ingrowth surfaces and extends into or preferably through the porous implant body substantially parallel to said forces, and wherein the supply channels (105) have cross sections larger than the cross sections of the porosity channels (103) and small enough for being bridgeable by spontaneous bone growth without additional bone growth enhancing material.

14. The system according to claim 13, wherein at least the porous implant body and the support frame consist of a same material and are manufactured in one single additive manufacturing process.

## Patentansprüche

1. Fenestrierter Knochenanker (210), der zur Verankerung in lebendem Knochengewebe eines menschlichen oder tierischen Patienten geeignet ist, wobei der Knochenanker (210) einen Schaft (212) mit einem proximalen Ende, einem distalen Ende, einer Längsachse und einer sich vom proximalen Ende zum distalen Ende erstreckenden Umfangsfläche aufweist, wobei der Schaft (212) ferner einen Längshohlraum (300), der sich in Richtung der Achse vom proximalen Ende zum distalen Ende erstreckt, und mindestens einen seitlichen Kanal (301) umfasst, der sich durch eine Wand (310) des Schafts (212) von der axialen Hohlraum (300) zur Umfangsfläche erstreckt, wobei die Wand (310) eine Wandstärke aufweist und der seitliche Kanal (301) einen Querschnitt mit einer axialen Länge (l) und einer Breite (w) aufweist, die kleiner ist als die axiale Länge, und wobei die Breite (w) in distaler Richtung zunimmt, und zusätzlich oder alternativ, die Wandstärke in zumindest ausgewählten Richtungen in Richtung des seitlichen Kanals graduell zunimmt, wobei der Knochenanker mehrere seitliche Kanäle (301) aufweist, die in einem gleichen ersten Abstand vom proximalen Ende des Schafts (212) angeordnet sind.

2. Knochenanker nach Anspruch 1, wobei alle seitlichen Kanäle (301) der mehreren seitlichen Kanäle (301) in regelmäßigen Abständen voneinander um die Umfangsfläche herum angeordnet sind.

3. Knochenanker nach Anspruch 1 oder 2, wobei der erste Abstand kleiner ist als ein zweiter Abstand zwischen dem seitlichen Kanal und dem distalen Ende des Schafts (212) und/oder wobei der erste Abstand kleiner ist als etwa die Hälfte einer Gesamtlänge des Schafts in axialer Richtung.

4. Knochenanker nach einem der Ansprüche 1 bis 3, wobei die Wandstärke zu jedem der seitlichen Kanäle (301) hin zunimmt und ein Querschnitt des Knochenankers (210) oder des Längshohlraums (300) durch die mehreren seitlichen Kanäle (301) eine lobulierte Form aufweist, wobei jedem der Kanäle ein Lappen zugeordnet ist

5. Knochenanker nach Anspruch 4, wobei in einer proximalen Richtung weg von der Vielzahl von seitlichen Kanälen (301) die lobulierte Form des Querschnitts des Knochenankers allmählich in eine kreisförmige Form übergeht.

6. Knochenanker nach einem der Ansprüche 1 bis 5, der ferner einen Kopf (211) aufweist und zum Fixieren einer Knochenplatte relativ zu Knochengewebe geeignet ist, wobei die Knochenplatte eine an den Knochenanker angepasste Durchgangsöffnung aufweist.

7. Knochenanker nach einem der Ansprüche 1 bis 6, der ferner an der Umfangsfläche des Schafts angeordnete Rückhaltestrukturen aufweist, wobei die Rückhaltestrukturen mindestens eines von einem Gewinde, Umfangsrippen, scharfen Kanten, Zähnen, Oberflächenrauhigkeit, hinterschnittenen Oberflächenstrukturen und einer die Osseointegration fördernden Oberflächenbeschichtung aufweisen.

8. Knochenanker nach einem der Ansprüche 1 bis 7, wobei an axialen Positionen des mindestens einen seitlichen Kanals (301) die Biegesteifigkeit des Ankers (210) in Richtung proximal graduell zunimmt.

9. Knochenanker nach einem der Ansprüche 1 bis 8, wobei über einen wesentlichen Abschnitt des seitlichen Kanals (301) der polare Flächenträgheitsmoment des Knochenankers (210) graduell in proximaler Richtung zunimmt.

10. Knochenanker nach einem der Ansprüche 1 bis 9, wobei eine durchschnittliche Umfangsbreite einer proximalen Hälfte des mindestens einen seitlichen Kanals wesentlich kleiner ist als eine durchschnittliche Umfangsbreite einer distalen Hälfte des seitlichen Kanals.

11. Chirurgisches System, das ein Implantat (100) mit mindestens einer Durchgangsöffnung, die eine Öffnungsachse definiert, und mindestens einen Knochenanker gemäß einem der Ansprüche 1 bis 10 aufweist und zum Fixieren des Implantats (100) relativ zu Knochengewebe eines menschlichen oder tierischen Patienten geeignet ist, wobei der Knochenanker einen Kopf und einen Schaft und eine Verankerungsachse aufweist, wobei die Durchgangsöffnung und der Knochenanker (210) so aufeinander abgestimmt sind, dass der Schaft (212) durch die Durchgangsöffnung hindurchgehen kann und der Kopf (211) von einer proximalen Fläche des Implantats (100) oder innerhalb der Durchgangsöffnung in einer Endposition gesichert werden kann, wobei das System zum Verriegeln des Ankers in der Endposition mindestens ein Verriegelungselement (200) aufweist , das zwischen einer entspannten Position, in der es aus einer allgemeinen Ebene eines Oberflächenabschnitts des Ankers (210) oder der Durchgangsöffnung herausragt, und einer elastisch vorgespannten Position, in der es weniger oder gar nicht aus dieser Ebene herausragt, bewegbar ist, wobei das Verriegelungselement (200) ein integraler Bestandteil des Knochenankers (210) oder des Implantats (100) ist und einen Teil des Oberflächenabschnitts und eines Volumens des Ankers oder des Implantats bildet, das unterhalb des Oberflächenabschnitts angeordnet ist, wobei ein Hohlraum (205) in dem Oberflächenabschnitt das Verriegelungselement (200) begrenzt und sich ferner unterhalb des Verriegelungselements (200) oder durch das Volumen erstreckt.

12. System nach Anspruch 11, wobei das Verriegelungselement (200) als elastischer Ausleger oder elastischer Biegebalken mit einer Länge und einer Breite ausgebildet ist, wobei das Verriegelungselement (200) ferner eine Rampe und eine Verriegelungsfläche aufweist, die benachbart zueinander angeordnet sind, wobei die Länge des Auslegers oder des Biegebalkens im Wesentlichen parallel zur Verankerungsachse oder zur Öffnungsachse verläuft und die Rampe und die Verriegelungsfläche benachbart zueinander über die Länge des Auslegers oder des Biegebalkens angeordnet sind, oder wobei das Verriegelungselement auf dem Schaft des Knochenankers angeordnet ist, wobei die Länge des Auslegers oder des Biegebalkens im Wesentlichen senkrecht zur Verankerungsachse oder zur Öffnungsachse verläuft und die Rampe und die Verriegelungsfläche über die Breite des Auslegers oder des Biegebalkens nebeneinander angeordnet sind, und wobei das Verriegelungselement am Kopf (211) des Knochenankers (210) angeordnet ist.

13. System nach Anspruch 11 oder 12, wobei das Implantat (100) ein lasttragendes Knochenimplantat ist, das geeignet ist, in einen menschlichen oder tierischen Patienten zwischen Oberflächen von lebenden Knochen oder Knochenfragmenten implantiert zu werden und geeignet ist, zwischen den Knochen oder Knochenfragmenten wirkende Kräfte zu übertragen, und das ferner geeignet ist, durch Knochenwachstum nach der Operation zwischen den Knochen oder Knochenfragmenten integriert zu werden, wobei das Knochenimplantat einen porösen Implantatkörper (102) und einen Stützrahmen aufweist, wobei der poröse Implantatkörper aufweist:
∘ gegenüberliegende Einwachsflächen (104), die an Oberflächen (101) der Knochen oder Knochenfragmente positioniert werden sollen,
o eine offene Porosität, die im gesamten porösen Implantatkörper (102) ein dreidimensionales Netzwerk aus Porositätskanälen (103) mit für das Einwachsen von Knochen geeigneten Abmessungen bildet, und
∘ eine Vielzahl von Versorgungskanälen (105), wobei jeder der Versorgungskanäle (105) eine Öffnung in mindestens einer der Einwachsflächen aufweist und sich in den porösen Implantatkörper oder vorzugsweise durch diesen hindurch im Wesentlichen parallel zu den genannten Kräften erstreckt, und wobei die Versorgungskanäle (105) Querschnitte aufweisen, die größer sind als die Querschnitte der Porositätskanäle (103) und klein genug, um durch spontanes Knochenwachstum ohne zusätzliches knochenwachstumsförderndes Material überbrückbar zu sein.

14. System nach Anspruch 13, wobei zumindest der poröse Implantatkörper und der Stützrahmen aus einem gleichen Material bestehen und in einem einzigen additiven Fertigungsprozess hergestellt sind.

## Revendications

1. Ancrage osseux fenêtré (210) adapté pour être ancré dans le tissu osseux vivant d'un patient humain ou animal, dans lequel l'ancrage osseux (210) comprend une tige (212) avec une extrémité proximale, une extrémité distale, un axe longitudinal et une surface circonférentielle s'étendant de l'extrémité proximale à l'extrémité distale, dans lequel la tige (212) comprend en outre une cavité longitudinale (300) s'étendant dans la direction de l'axe depuis l'extrémité proximale vers l'extrémité distale et au moins un canal latéral (301) s'étendant à travers une paroi (310) de la tige (212) depuis la cavité axiale (300) jusqu'à la surface circonférentielle, la paroi (310) ayant une épaisseur de paroi et le canal latéral (301) ayant une section transversale avec une longueur axiale (l) et une largeur (w) inférieure à la longueur axiale, et dans lequel ladite largeur (w) augmente dans une direction distale et, en outre ou en variante, ladite épaisseur de paroi augmente progressivement dans au moins certaines directions sélectionnées vers le canal latéral, dans lequel l'ancrage osseux comprend une pluralité de canaux latéraux (301) disposés à une même première distance de l'extrémité proximale de la tige (212).

2. L'ancrage osseux selon la revendication 1, dans lequel tous les canaux latéraux (301) de la pluralité de canaux latéraux (301) sont régulièrement espacés les uns des autres autour de la surface circonférentielle.

3. L'ancrage osseux selon la revendication 1 ou 2, dans lequel la première distance est inférieure à une deuxième distance entre le canal latéral et l'extrémité distale de la tige (212), et/ou dans lequel la première distance est inférieure à environ la moitié d'une longueur axiale totale de la tige.

4. L'ancrage osseux selon l'une quelconque des revendications 1 à 3, dans lequel l'épaisseur de paroi augmente vers chacun des canaux latéraux (301) et une section transversale de l'ancrage osseux (210) ou de la cavité longitudinale (300) à travers la pluralité de canaux latéraux (301) a une forme lobée ayant un lobe associé à chacun des canaux.

5. L'ancrage osseux selon la revendication 4, dans lequel, dans une direction proximale s'éloignant de la pluralité de canaux latéraux (301), la forme lobée de la section transversale de l'ancrage osseux se transforme progressivement en une forme circulaire.

6. L'ancrage osseux selon l'une quelconque des revendications 1 à 5, comprenant en outre une tête (211) et étant adapté pour fixer une plaque osseuse par rapport au tissu osseux, dans lequel la plaque osseuse comprend une ouverture traversante adaptée à l'ancrage osseux.

7. L'ancrage osseux selon l'une quelconque des revendications 1 à 6, comprenant en outre des structures de retenue disposées sur la surface circonférentielle de la tige, dans lequel les structures de retenue comprennent au moins l'un d'un filetage, de nervures circonférentielles, d'arêtes vives, de dents, d'une rugosité de surface, de structures de surface en contre-dépouille et d'un revêtement de surface augmentant l'ostéointégration.

8. L'ancrage osseux selon l'une quelconque des revendications 1 à 7, dans lequel, au niveau des positions axiales du au moins un canal latéral (301), la rigidité de l'ancrage (210) à la flexion augmente progressivement vers la partie proximale.

9. L'ancrage osseux selon l'une quelconque des revendications 1 à 8, dans lequel, sur une partie substantielle du canal latéral (301), le moment d'inertie polaire de l'ancrage osseux (210) augmente progressivement vers la partie proximale.

10. L'ancrage osseux selon l'une quelconque des revendications 1 à 9, dans lequel une largeur circonférentielle moyenne d'une moitié proximale du au moins un canal latéral est sensiblement plus petite qu'une largeur circonférentielle moyenne d'une moitié distale du canal latéral.

11. Système chirurgical comprenant un implant (100) avec au moins une ouverture traversante définissant un axe d'ouverture et au moins une ancre osseuse définie selon l'une quelconque des revendications 1 à 10 et adaptée pour fixer l'implant (100) par rapport au tissu osseux d'un patient humain ou animal, l'ancre osseuse comprenant une tête et une tige et un axe d'ancrage, l'ouverture traversante et l'ancrage osseux (210) étant adaptés l'un à l'autre pour que la tige (212) puisse passer à travers l'ouverture traversante et que la tête (211) puisse être retenue par une surface proximale de l'implant (100) ou à l'intérieur de l'ouverture traversante dans une position finale, dans lequel, pour bloquer l'ancrage dans ladite position finale, le système comprend en outre au moins un élément de blocage (200) pouvant être déplacé entre une position détendue dans laquelle il fait saillie à partir d'un niveau général d'une partie de surface de l'ancrage (210) ou de l'ouverture traversante et une position tendue élastiquement dans laquelle il fait moins ou pas du tout saillie à partir dudit niveau, dans lequel l'élément de blocage (200) est une partie intégrante de l'ancrage osseux (210) ou de l'implant (100), constitue une partie de ladite partie de surface et d'une masse de l'ancrage ou de l'implant située sous ladite partie de surface, dans lequel un vide (205) dans la partie de surface délimite l'élément de blocage (200) et s'étend en outre sous l'élément de blocage (200) ou à travers ladite masse.

12. Le système selon la revendication 11, dans lequel l'élément de blocage (200) est formé comme un cantilever élastique ou une poutre de flexion élastique ayant une longueur et une largeur, dans lequel l'élément de blocage (200) comprend en outre une rampe et une surface de blocage disposées de manière adjacente l'une à l'autre, dans lequel la longueur du cantilever ou de la poutre de flexion s'étend sensiblement parallèlement à l'axe de l'ancrage ou à l'axe d'ouverture et la rampe et la surface de blocage sont disposées de manière adjacente l'une à l'autre sur la longueur du cantilever ou de la poutre de flexion, ou dans lequel l'élément de blocage est disposé sur la tige de l'ancrage osseux, dans lequel la longueur du cantilever ou de la poutre de flexion s'étend sensiblement perpendiculairement à l'axe d'ancrage ou à l'axe d'ouverture, et la rampe et la surface de blocage sont disposées de manière adjacente l'une à l'autre sur la largeur du cantilever ou de la poutre de flexion, et dans lequel l'élément de blocage est disposé sur la tête (211) de l'ancrage osseux (210).

13. Le système selon la revendication 11 ou 12, dans lequel l'implant (100) est un implant osseux porteur adapté pour être implanté chez un patient humain ou animal entre des surfaces d'os vivants ou de fragments d'os, adapté pour transmettre des forces agissant entre les os ou les fragments d'os, et adapté pour être intégré entre les os ou les fragments d'os par croissance osseuse après une intervention chirurgicale, l'implant osseux comprenant un corps d'implant poreux (102) et un cadre de support, dans lequel le corps d'implant poreux comprend :
o des surfaces de croissance interne opposées (104) destinées à être positionnées contre les surfaces (101) des os ou des fragments d'os,
o une porosité ouverte constituant, dans tout le corps d'implant poreux (102), un réseau tridimensionnel de canaux de porosité (103) de dimensions adaptées à la croissance osseuse, et
o une pluralité de canaux d'alimentation (105), dans lequel chacun des canaux d'alimentation (105) a une embouchure dans au moins l'une des surfaces de croissance interne et s'étend dans ou de préférence à travers le corps d'implant poreux sensiblement parallèlement auxdites forces, et dans lequel les canaux d'alimentation (105) ont des sections transversales plus grandes que les sections transversales des canaux de porosité (103) et suffisamment petites pour être pontables par la croissance osseuse spontanée sans matériau supplémentaire augmentant la croissance osseuse.

14. Le système selon la revendication 13, dans lequel au moins le corps d'implant poreux et le cadre de support consistent en un même matériau et sont fabriqués en un seul processus de fabrication additive.
